# EUROPEAN PATENT SPECIFICATION

(11) **EP 3 110 335 B1**
(45) Date of publication and mention of the grant of the patent: **07.09.2022**
(21) Application number: 15711574.2
(22) Date of filing: 16.02.2015
(51) Int. Cl.: A61B 8/08, A61B 8/00, G06T 7/00, G06T 7/30, G06T 7/11, G06T 7/246

(54) **ZONE VISUALIZATION FOR ULTRASOUND-GUIDED PROCEDURES**
ZONENVISUALISIERUNG FÜR ULTRASCHALLGEFÜHRTE EINGRIFFE
VISUALISATION DE ZONE POUR PROCÉDURES ÉCHOGUIDÉES

(30) Priority: 28.02.2014 US 201461945897 P
(43) Date of publication of application: 04.01.2017
(73) Proprietor: Koninklijke Philips N.V., 5656 AG Eindhoven (NL)
(72) Inventor: YAN, Pingkun, NL-5656 AG Eindhoven (NL); KRUECKER, Jochen, NL-5656 AG Eindhoven (NL)
(74) Representative: Philips Intellectual Property & Standards
(86) International application number: PCT/IB2015/051135
(87) International publication number: WO 2015/128772

(56) References cited:
- WO-A1-2013/153506
- US-A1- 2012 172 724
- US-A1- 2013 237 811
- US-A1- 2013 324 841

## Description

The present invention generally relates to automatic location of specific zones of an anatomical structure for visual guidance during an ultrasound-guided procedure (e.g., a prostate biopsy). The present invention specifically relates to zone labeling of a three-dimensional ("3D") model of the anatomical structure as a basis for visualizing guidance through the zones during an ultrasound-guided procedure.

A medical image registration of a preoperative anatomical image with an intraoperative anatomical image has been utilized to facilitate image-guided interventional/surgical/diagnostic procedures. The main goal for the medical image registration is to calculate a geometrical transformation that aligns the same or different view of the same anatomical structure within the same or different imaging modality.

More particularly, prostate cancer affects one in six men in the western world, and it is the second leading cause of cancer death in American men. Transrectal ultrasound ("TRUS")-guided systematic biopsy with different schemes (e.g., sextant, extended 12 core, etc.) are considered to be the standard of care in clinical practice. However, as two-dimensional ("2D") ultrasound imaging is usually used, the field of view is limited. Furthermore, due to the lack of landmarks inside the prostate under ultrasound imaging, ultrasound based prostate imaging requires a significant amount of training and experience to precisely navigate to the desired location for biopsy.

Of importance, according to the urological literature on prostate biopsy, the cancer locations are not uniformly distributed inside the prostate gland. The research shows that there are some high risk areas inside the gland with higher possibilities to detect cancer. Known systematic biopsy schemes are designed in special ways to cover those high risk areas to achieve maximal cancer detection rate with certain number of biopsies. However, due to the limitations of the current ultrasound imaging guidance, there are trends that some zones (e.g., horns of the prostate gland) tend to be missed in many cases. It may result in higher false negative biopsy rates and some cancer cases may be missed.

In summary, it is known that some high risk areas of the prostate can be significantly undersampled during biopsy due to the limitation of the imaging guidance technique. While such limitations may be compensated by the experience of physicians, the present invention provides a systematic technical approach to improve the outcome of biopsy consistently by assisting physicians with automatic zone identification. More particularly, an ultrasound model of an anatomical structure is labeled to two (2) or more procedurally-defined zones derived from a scheme designed for optimizing an ultrasound-guided procedure on the anatomical structure. For example, an ultrasound model of prostate may be labeled by procedurally-defined zones that are associated with a known scheme for an ultrasound-guided biopsy sampling of the gland, particularly a scheme considered to be the standard of care in clinical practice (e.g., sextant, extended 12 core, saturation sampling, anterior sampling etc.).

United States Patent Application US 2012/172724 describes an intracardiac imaging system configured to display electrode visualization elements within an intracardiac echocardiography image where the electrode visualization elements represent intracardiac electrodes in close proximity to the plane of the image. The system further allows cross sections of tissue structures embodied in intracardiac echocardiography images to be modeled within a visualization, navigation, or mapping system when automatically segmented to generate shell elements for modifying the modeled tissue structures.

One form of the present invention is a system according to claim 1 and a method according to claim 9.

The foregoing form and other forms of the present invention as well as various features and advantages of the present invention will become further apparent from the following detailed description of various embodiments of the present invention read in conjunction with the accompanying drawings. The detailed description and drawings are merely illustrative of the present invention rather than limiting, the scope of the present invention being defined by the appended claims.
FIG. 1 illustrates zone labeled ultrasound volumes in accordance with the present invention.
FIG. 2 illustrates a flowchart representative of an exemplary embodiment of an automatic visualization method in accordance with the present invention.
FIG. 3 illustrates an exemplary implementation of the flowchart illustrated in FIG. 2.

For purposes of the present invention, the term a "procedurally-defined zone" is broadly defined as zones of an anatomical structure derived from a scheme designed for optimizing an ultrasound-guided procedure on the anatomical structure. For example, an ultrasound model of prostate may be labeled by procedurally-defined zones that are associated with a known or proposed scheme for an ultrasound-guided biopsy sampling of the gland, particularly a scheme considered to be the standard of care in clinical practice (e.g., sextant, extended 12 core, saturation sampling, anterior sampling etc.).

Also, for purposes of the present invention, the terms "tracking", "reconstruction", "segmentation" and "registration" as well as related terms are to be broadly interpreted as known in the art of the present invention.

In practice, the present invention applies to any anatomical regions (e.g., head, thorax, pelvis, etc.) and anatomical structures (e.g., bones, organs, circulatory system, digestive system, etc.).

To facilitate an understanding of the present invention, exemplary embodiments of the present invention will be provided herein directed to automatic zone visualization of ultrasound imaging of a prostrate. Nonetheless, those having ordinary skill in the art will appreciate how to execute automatic zone visualization of ultrasound imaging for all anatomical regions and structures therein.

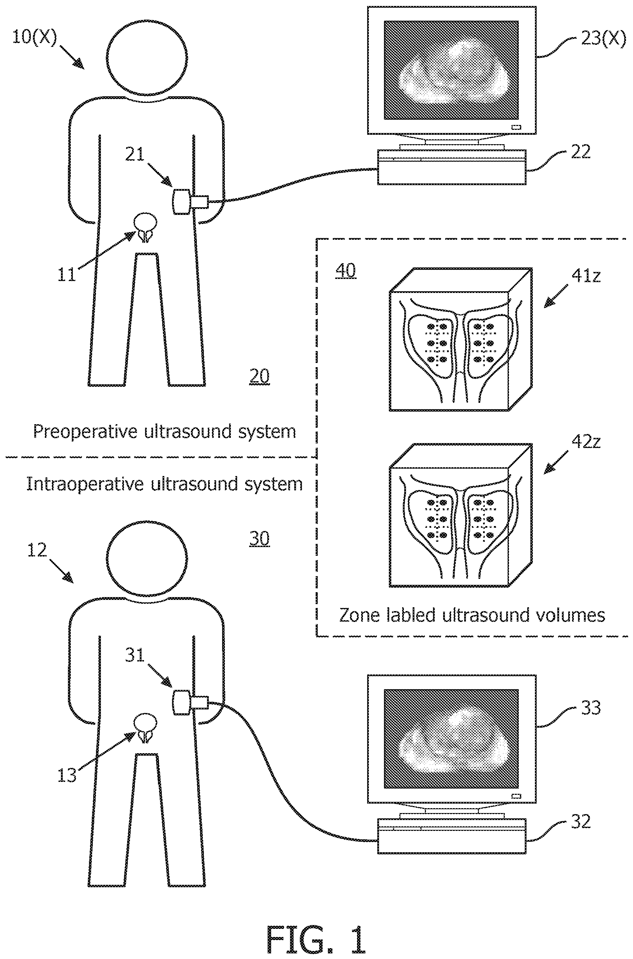

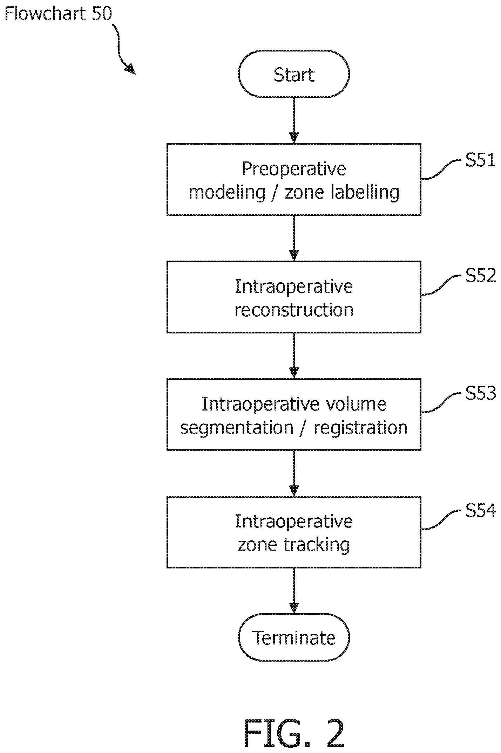

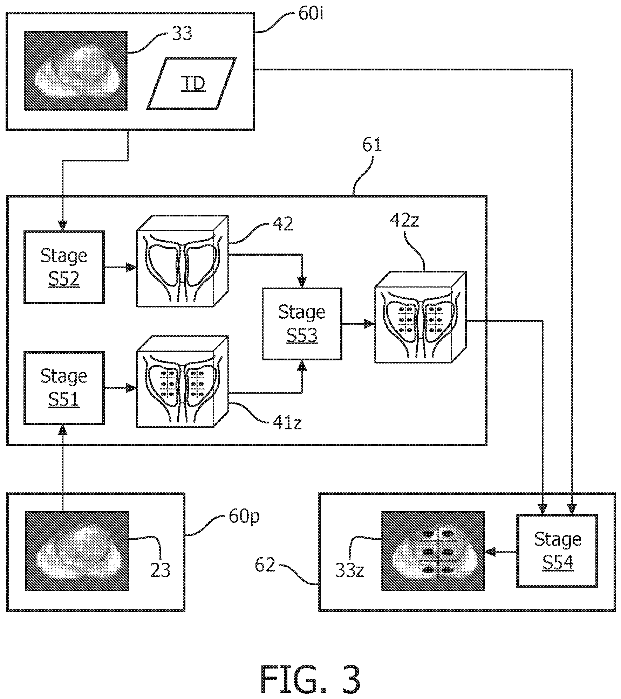

Referring to FIG. 1, a preoperative ultrasound system 20 employs a 2D ultrasound probe 21 and an ultrasound imaging workstation 22 to generate a stream of ultrasound images 23 of an anatomical tissue of a prostate 11 of a subject 10 as subject 10 is being scanned by 2D ultrasound probe 21. As will be further described in connection with FIGS. 2 and 3, preoperative ultrasound system 20 is utilized to scan an X number of subjects 10 to acquire an X number of ultrasound volumes of prostate 11 reconstructed from the ultrasound images to thereby build a volume model 41z of prostate 11 labeled with procedurally-defined zones as symbolically shown by the matrix of dots therein. Alternatively, system 20 may employ a 3D ultrasound probe (not shown).

An intraoperative ultrasound system 30 employs a 2D ultrasound probe 31 and a ultrasound imaging workstation 32 to generate a stream of ultrasound images 33 of an anatomical tissue of a prostate 13 of a patient 12 as patient 12 is being scanned by 2D ultrasound probe 31. As will be further described in connection with FIGS. 2 and 3, intraoperative ultrasound system 20 is utilized to scan patient 12 to acquire an ultrasound volume 42z of prostate 13 and to track a registration of zone labeled volume model 41z to ultrasound volume 42z to thereby label ultrasound volume 42z with the procedurally-defined zones as symbolically shown by the matrix of dots therein.

Please note the images of the zone labeled prostate within model 41 and volume 42 are not intended to be anatomically correct, but serves only as a simplified example of a zone labeled prostate for purposes of facilitating a description of an automatic zone visualization of the present invention based on zone labeled model 41z and volume 42z, which will now be provided herein.

Referring to FIGS. 2 and 3, the workflow of a flowchart 50 (FIG. 2) encompasses three (3) major aspects (FIG. 3) including various resource inputs 60, a tracked image registration 61, and a tracked output visualization 62.

Generally, a preoperative resource 60p includes a stream of scanned ultrasound images 23 of prostate 11 for each subject 10 (FIG. 1) (or alternatively a 3D ultrasound image of prostate 11 of one or more subjects 10). Intraoperative resource 60i includes a stream of scanned ultrasound images 33 of prostate 13 of patient 12 (FIG. 1) (or alternatively a 3D ultrasound image of prostate 13 of patient 12) and tracking data TD of 2D ultrasound probe 31 (FIG. 1) as patient 12 is scanned. Output visualization 62 symbolically shows the zones on a real-time stream of ultrasound image 33 in real time in accordance with a visualization strategy for differentiating the zones (e.g., color coding, text labels and/or audio feedback).

Between inputs 60 and output 62, image registration 61 includes various processes for building prostate volume model 41z, tracking a reconstruction of a volume image 42 from ultrasound image stream 33, mapping a segmented prostate from volume image 42 to volume model 41z. By doing these aspects, the zones can be mapped to the real time ultrasound streaming 33z by using the device tracking data TD.

More particularly, ultrasound system 30 (FIG. 1) may stream ultrasound images 33 by either capturing the screen display of ultrasound imaging workstation 32, or directly from an output port of ultrasound imaging workstation 32 in an internal transfer mode. Alternatively to 2D ultrasound probe 31, a 3D ultrasound probe can be used to obtain a 3D image of the prostate without the need for reconstructing the volume from 2D images.

Ultrasound system 30 also employs or cooperates with a tracking system to obtain a pose and position of 2D ultrasound probe 31 in real time for mapping ultrasound image stream 33 (or 3D image) to the 3D patient space. The pose and position of ultrasound probe 31 may be obtained by using any known device tracking technique (e.g., electromagnetic tracking or optical sensor tracking)

Flowchart 50 has four main stages S51-S54. A stage S51 of flowchart 50 encompasses workstation 22 (FIG. 1) building prostate volume model 41z with procedurally-defined zones. Specifically, a statistical shape model of the prostate is built by workstation 22 using a training data set having shapes obtained retrospectively from subjects 10 (FIG. 1). For each shape, corresponding zones of interest are labeled. The zones are procedurally-defined zones associated with a known or proposed scheme for an ultrasound-guided biopsy sampling of the gland, particularly a scheme considered to be the standard of care in clinical practice (e.g., sextant, extended 12 core, etc.). By applying statistical shape analysis (e.g., principal component analysis), prostate volume model 41z with labeled procedurally-defined zones is built by workstation 22.

In one embodiment, a preferred biopsy sampling scheme is added to the prostate volume model 41z by identifying a number *N* of locations in the prostate (typically N ≥ 10). Each location can be identified as a geometrical object (e.g., a point, a small sphere or other simple shape (e.g. ellipsoid) in prostate volume model 41z. These locations can later serve to guide the systematic sampling of the prostate according to the desired scheme.

A stage S52 of flowchart 50 encompasses workstation 32 (FIG. 1) reconstructing an ultrasound volume 42. Specifically, as ultrasound probe 31 is being tracked while scanning prostate 13 of patient 12, the pose and position of ultrasound probe 31 are known. By using this tracking data TD, ultrasound frames of stream 33 are transformed to the 3D patient space. By performing a sweep through the whole gland to obtain ultrasound image stream 33 covering the entire prostate 13, ultrasound volume 42 containing the prostate is reconstructed. Alternatively, if a 3D probe is used for acquisition, reconstruction of stage S52 may be omitted since the ultrasound workstation 32 provides the 3D volume image directly. Nonetheless, the 3D probe will be tracked to transform ultrasound volume 42 to 3D patient space.

A stage S53 of flowchart 50 encompasses workstation 32 segmenting the prostate from ultrasound volume 42 and registering prostate volume model 41z with the segmented prostate. Specifically, a main purpose of performing segmentation is to assist registering the prostate volume model 41z to ultrasound volume 42, as it is very challenging to directly map prostate volume model 41z to ultrasound image stream 33. The segmentation may be performed in two (2) ways. The first option is to segment the ultrasound sweep data frame by frame. The obtained 2D segmentation sequences are then mapped to 3D space using the same transformations as in the reconstruction stage S52 to get the 3D segmentation. Alternatively, the reconstructed ultrasound volume 42 is segmented directly in 3D space by using a model based approach. If the prostate boundary in ultrasound volume 42 is not as clear as in the 2D ultrasound image stream 33, the two (2) options maybe combined to achieve better segmentation performance.

Once the reconstructed ultrasound volume 42 is segmented, a surface based registration method (e.g., iterative closest points based registration) is applied to register prostate volume model 41z with the segmented prostate of reconstructed ultrasound volume 42 to yield a zone labeled ultrasound volume 42z. With this registration, procedurally zones labeled in prostate volume model 42z are mapped to the patient space. With the real time tracking information available, the zones may be transformed to ultrasound image stream 33.

A stage S54 of flowchart 50 encompasses workstation 32 displaying a zone visualization in real time. Specifically, once the zones are mapped to ultrasound image stream 33, procedurally-defined zone(s) can be visualized over an ultrasound image 33z when being intersected. The intersected zone(s) are highlighted with a zone label displayed. In addition, different visualized zones may be differentiated with color coding, text labels, or audio feedback. For example, while a set of zones are being intersected by a ultrasound image 33z, the intersection areas are shown in each corresponding color or label with or without audio feedback. As an addition or alternative approach, the locations of the biopsy sampling scheme are visualized jointly with ultrasound image 33z. This helps the user to adjust ultrasound probe 31 look direction until the biopsy path is aligned with the locations of the sampling scheme.

Flowchart 50 is terminated upon completion of the procedure.

In an alternative embodiment of flowchart 50, stage S51 and the registration of stage S53 may be omitted whereby prostate volume model 41z may be defined using reconstructed ultrasound volume 42 in lieu of compounding a model using X number of prior subjects. Specifically, the procedurally-defined zones are created based on geometric sub-division of an intra-procedural segmentation of reconstructed ultrasound volume 42. Examples of such sub-division include, but are not limited to, (1) dividing the intra-procedural segmentation of reconstructed ultrasound volume 42 in two (2) halves along the mid-sagittal plane and thus creating a "left" and "right" zone, and (2) dividing in the intra-procedural segmentation of reconstructed ultrasound volume 42 thirds using axial cut-planes, thus creating base/mid-gland/axial zones.

Referring to FIGS. 1-3, those having ordinary skill in the art will appreciate numerous benefits of the present invention including, but not limited to, automatic zone visualization of ultrasound-imaged guidance of anatomical structures.

While various embodiments of the present invention have been illustrated and described, it will be understood by those skilled in the art that the embodiments of the present invention as described herein are illustrative, and various changes and modifications may be made without departing from the true scope of the present invention. In addition, many modifications may be made to adapt the teachings of the present invention without departing from its central scope. Therefore, it is intended that the present invention not be limited to the particular embodiments disclosed as the best mode contemplated for carrying out the present invention, but that the present invention includes all embodiments falling within the scope of the appended claims.

## Claims

1. A system for automatic zone visualization, the system comprising:
an ultrasound probe (31) operable to scan an anatomical region comprising a prostate; and
an ultrasound imaging workstation (32) operably connected to the ultrasound probe (31) to track a generation of an ultrasound volume (42) of the prostate within a patient space responsive to a scan of the anatomical region by the ultrasound probe (31),
wherein the ultrasound imaging workstation (32) is operable to track a labeling of procedurally-defined zones of the prostate within the ultrasound volume (42) derived from an ultrasound volume model (41) of the prostate labeled with the procedurally-defined zones, and
wherein the procedurally-defined zones within the ultrasound volume (42) facilitate an ultrasound-guided visualization of the prostate according to a biopsy sampling scheme, which is added to the ultrasound volume model of the prostate (41) by identifying a number of N locations in the prostate as geometrical objects which serve to guide a systematic sampling of the prostate.

2. The system of claim 1, wherein the ultrasound imaging workstation (32) maps ultrasound volume model (41) to a tracked generation of the ultrasound volume (42).

3. The system of claim 1, wherein the ultrasound imaging workstation (32) maps ultrasound volume model (41) to a segmentation of the prostate from a tracked generation of the ultrasound volume (42).

4. The system of claim 1, wherein the ultrasound imaging workstation (32) generates the ultrasound volume model (41) as a geometric sub-division model of a segmentation of the prostate within the ultrasound volume (42).

5. The system of claim 1,
wherein the ultrasound probe (31) is further operable to guide a visualization of at least one of the procedurally-defined zones of the prostate;
wherein the ultrasound imaging workstation (32) is operably connected to the ultrasound probe (31) to generate a ultrasound image (33) responsive to the ultrasound probe (31) guiding a visualization of the at least one of the procedurally-defined zones of the prostate; and
wherein the ultrasound image (33) visualizes the at least one of the procedurally-defined zones derived from the zone labeled ultrasound volume (42).

6. The system of claim 5, wherein the ultrasound imaging workstation (32) tracks a generation of the ultrasound image (33) within the patient space and zone labels the at least one of the procedurally-defined zones on the ultrasound image (33) as a function of a tracking intersection of the ultrasound image (33) and the ultrasound volume (42).

7. The system of claim 5, wherein the ultrasound imaging workstation (32) differentiates at least two procedurally-defined zones visualized within the ultrasound image (33).

8. The system of claim 7, wherein a differentiation of the at least two procedurally-defined zones visualized within the ultrasound image (33) includes at least one of color coding, text labeling and audio feedback.

9. A computer-implemented method for automatic zone visualization, the method comprising:
generating an ultrasound volume (42) of a prostate derived from an ultrasound scan of an anatomical region; and
tracking a labeling of procedurally-defined zones of the prostate within the ultrasound volume (42) derived from an ultrasound volume model (41) of the prostate labeled with the procedurally-defined zones,
wherein the procedurally-defined zones within the ultrasound volume (42) facilitate an ultrasound-guided visualization of the prostate according to a biopsy sampling scheme, which is added to the ultrasound volume model of the prostate (41) by identifying a number of N locations in the prostate as geometrical objects which serve to guide a systematic sampling of the prostate.

10. The method of claim 9, wherein the tracking of the labeling of procedurally-defined zones of the prostate within the ultrasound volume (42) includes:
mapping the ultrasound volume model (41) to a tracked generation of the ultrasound volume (42).

11. The method of claim 9, wherein the tracking of the labeling of procedurally-defined zones of the prostate within the ultrasound volume (42) includes:
mapping the ultrasound volume model (41) to a segmentation of the prostate from a tracked generation of the ultrasound volume (42).

12. The method of claim 9, wherein the ultrasound volume model (41) is a geometric sub-division model of a segmentation of the prostate within the ultrasound volume (42).

13. The method of claim 9, further comprising:
generating a ultrasound image (33) responsive to an ultrasound probe (31) guiding a visualization of at least one of the procedurally-defined zones of the prostate,
wherein the ultrasound image (33) visualizes the at least one of the procedurally-defined zones derived from the zone labeled ultrasound volume (42).

14. The method of claim 13,
wherein the generation of the ultrasound image (33) is tracked within the patient space and
wherein the at least one of the procedurally-defined zones is zone labeled on the ultrasound image (33) as a function of a tracking intersection of the ultrasound image (33) and the ultrasound volume (42).

15. The method of claim 14, wherein at least two procedurally-defined zones are differentiated within the ultrasound image (33).

## Patentansprüche

1. System zur automatischen Zonendarstellung, wobei das System Folgendes umfasst: eine Ultraschallsonde (31), die so betrieben werden kann, dass sie einen anatomischen Bereich abtastet, der eine Prostata umfasst; und
eine bildgebende Ultraschall-Station (32), die funktionsfähig mit der Ultraschallsonde (31) verbunden ist, um eine Erzeugung eines Ultraschallvolumens (42) der Prostata innerhalb eines Patientenraums in Reaktion auf eine Abtastung des anatomischen Bereichs durch die Ultraschallsonde (31) Zu verfolgen,
wobei die bildgebende Ultraschall-Station (32) so betrieben werden kann, dass sie eine Kennzeichnung von verfahrensmäßig definierten Zonen der Prostata innerhalb des Ultraschallvolumens (42) verfolgt, das von einem Ultraschall-Volumenmodell (41) der Prostata abgeleitet ist, das mit den verfahrensmäßig definierten Zonen gekennzeichnet ist, und wobei die verfahrensmäßig definierten Zonen innerhalb des Ultraschallvolumens (42) eine ultraschallgeführte Visualisierung der Prostata gemäß einem Biopsie-Probenentnahmeschema erleichtern, das dem Ultraschall-Volumenmodell (41)der Prostata hinzugefügt wird, indem eine Anzahl von N Stellen in der Prostata als geometrische Objekte identifiziert wird, die dazu dient, eine systematische Probenentnahme der Prostata zu leiten.

2. System nach Anspruch 1, wobei die bildgebende Ultraschall-Station (32) das Ultraschallvolumenmodell (41) auf eine nachverfolgte Erzeugung des Ultraschallvolumens (42) abbildet.

3. System nach Anspruch 1, wobei die bildgebende Ultraschall-Station (32) ein Ultraschall-Volumenmodell (41) auf eine Segmentierung der Prostata aus einer nachverfolgten Erzeugung des UltraschallVolumens (42) abbildet.

4. System nach Anspruch 1, wobei die bildgebende Ultraschall-Station (32) das Ultraschallvolumenmodell (41) als geometrisches Unterteilungsmodell einer Segmentierung der Prostata innerhalb des Ultraschallvolumens (42) erzeugt.

5. Das System nach Anspruch 1,
wobei die Ultraschallsonde (31) ferner so betrieben werden kann, dass sie eine Visualisierung von mindestens einer der verfahrensmäßig definierten Zonen der Prostata leitet; wobei die bildgebende Ultraschall-Station (32) betriebsmäßig mit der Ultraschallsonde (31) verbunden ist, um ein Ultraschallbild (33) zu erzeugen, das auf die Ultraschallsonde (31) reagiert, die eine Visualisierung der mindestens einen der verfahrensmäßig definierten Zonen der Prostata leitet; und wobei das Ultraschallbild (33) die mindestens eine der verfahrensmäßig definierten Zonen visualisiert, die von dem zonenmarkierten Ultraschallvolumen (42) abgeleitet ist.

6. System nach Anspruch 5, wobei die bildgebende Ultraschall-Station (32) eine Erzeugung des Ultraschallbildes (33) innerhalb des Patientenraums verfolgt und die mindestens eine der verfahrensmäßig definierten Zonen auf dem Ultraschallbild (33) in Abhängigkeit von einem Verfolgungsschnittpunkt des Ultraschallbildes (33) und des Ultraschallvolumens (42) kennzeichnet.

7. System nach Anspruch 5, wobei die bildgebende Ultraschall-Station (32) mindestens zwei prozedural definierte Zonen unterscheidet, die in dem Ultraschallbild (33) sichtbar sind.

8. System nach Anspruch 7, wobei eine Unterscheidung der mindestens zwei verfahrensmäßig definierten Zonen, die im Ultraschallbild (33) visualisiert werden, mindestens eine der folgenden Möglichkeiten umfasst: Farbcodierung, Textbeschriftung und Audio-Feedback.

9. Computer-implementiertes Verfahren zur automatischen Zonendarstellung, wobei das Verfahren Folgendes umfasst: Erzeugen eines Ultraschallvolumens (42) einer Prostata, das von einem Ultraschallscan eines anatomischen Bereichs abgeleitet ist und Verfolgen einer Kennzeichnung von verfahrensmäßig definierten Zonen der Prostata innerhalb des Ultraschallvolumens (42), das von einem Ultraschallvolumenmodell (41) der Prostata abgeleitet ist, das mit den verfahrensmäßig definierten Zonen gekennzeichnet ist, wobei die verfahrensdefinierten Zonen innerhalb des Ultraschallvolumens (42) eine ultraschallgeführte Visualisierung der Prostata gemäß einem Biopsie-Probenentnahmeschema erleichtern, das dem Ultraschall-Volumenmodell der Prostata (41) hinzugefügt wird, indem eine Anzahl von N Stellen in der Prostata als geometrische Objekte identifiziert wird, die dazu dient, eine systematische Probenentnahme der Prostata zu leiten.

10. Verfahren nach Anspruch 9, wobei die Verfolgung der Markierung von verfahrensmäßig definierten Zonen der Prostata innerhalb des Ultraschallvolumens (42) umfasst:
Abbildung des Ultraschallvolumenmodells (41) auf eine nachverfolgte Generation des Ultraschallvolumens (42).

11. Verfahren nach Anspruch 9, wobei die Nachverfolgung der Kennzeichnung von verfahrensmäßig definierten Zonen der Prostata innerhalb des Ultraschallvolumens (42) Folgendes umfasst: Abbilden des Ultraschallvolumenmodells (41) auf eine Segmentierung der Prostata aus einer nachverfolgten Erzeugung des Ultraschallvolumens (42).

12. Verfahren nach Anspruch 9, wobei das Ultraschallvolumenmodell (41) ein geometrisches Unterteilungsmodell einer Segmentierung der Prostata innerhalb des
Ultraschallvolumens(42) ist.

13. Verfahren nach Anspruch 9, ferner Folgendes umfassend: Erzeugen eines Ultraschallbildes (33), das auf eine Ultraschallsonde (31) anspricht, die eine Visualisierung von mindestens einer der verfahrensmäßig definierten Zonen der Prostata leitet, wobei das Ultraschallbild (33) die mindestens eine der verfahrensmäßig definierten Zonen visualisiert, die von dem zonenmarkierten Ultraschallvolumen (42) abgeleitet ist.

14. Verfahren nach Anspruch 13, wobei die Erzeugung des Ultraschallbildes (33) innerhalb des Patientenbereichs nachverfolgt wird und
wobei mindestens eine der verfahrensmäßig definierten Zonen auf dem Ultraschallbild (33) in Abhängigkeit von einem Verfolgungsschnittpunkt des Ultraschallbildes (33) und des Ultraschallvolumens (42) zonenmarkiert ist.

15. Verfahren nach Anspruch 14, wobei im Ultraschallbild (33) mindestens zwei prozedural definierte Zonen unterschieden werden.

## Revendications

1. Système de visualisation automatique de zone, le système comprenant: une sonde à ultrasons (31) pouvant fonctionner pour balayer une région anatomique comprenant une prostate; et
une station de travail d'imagerie par ultrasons (32) connectée de manière opérationnelle à la sonde à ultrasons (31) pour suivre une génération d'un volume ultrasonore (42) de la prostate à l'intérieur d'un espace patient en réponse à un balayage de la région anatomique par la sonde à ultrasons (31),
dans lequel la station de travail d'imagerie par ultrasons (32) est utilisable pour suivre un étiquetage de zones de la prostate définies de manière procédurale dans le volume ultrasonore (42) dérivé d'un modèle de volume ultrasonore (41) de la prostate étiqueté avec les zones définies de manière procédurale, et
dans lequel les zones définies de manière procédurale à l'intérieur du volume ultrasonore (42) facilitent une visualisation guidée par ultrasons de la prostate selon un schéma d'échantillonnage par biopsie, qui est ajouté au modèle volumique ultrasonore de la prostate (41) en identifiant un certain nombre de N emplacements dans la prostate comme objets géométriques qui servent à guider un échantillonnage systématique de la prostate.

2. Système de la revendication 1, dans lequel la station de travail d'imagerie ultrasonore (32) mappe le modèle de volume ultrasonore (41) à une génération suivie du volume ultrasonore (42).

3. Système selon la revendication 1, dans lequel la station de travail d'imagerie ultrasonore (32) mappe le modèle de volume ultrasonore (41) sur une segmentation de la prostate à partir d'une génération suivie du volume ultrasonore (42).

4. Système selon la revendication 1, dans lequel la station de travail d'imagerie ultrasonore (32) génère le modèle de volume ultrasonore (41) en tant que modèle de subdivision géométrique d'une segmentation de la prostate dans le volume ultrasonore (42).

5. Système selon la revendication 1,
dans lequel la sonde à ultrasons (31) est en outre utilisable pour guider une visualisation d'au moins une des zones de la prostate définies de manière procédurale;
dans lequel la station de travail d'imagerie à ultrasons (32) est connectée de manière opérationnelle à la sonde à ultrasons (31) pour générer une image à ultrasons (33) en réponse à la sonde à ultrasons (31) guidant une visualisation de l'au moins une des zones de la prostate définies de manière procédurale; et dans lequel l'image ultrasonore (33) visualise l'au moins une des zones définies de manière procédurale dérivée du volume ultrasonore étiqueté par zone (42).

6. Système selon la revendication 5, dans lequel la station de travail d'imagerie ultrasonore (32) suit une génération de l'image ultrasonore (33) à l'intérieur de l'espace du patient et étiquette par zone l'au moins une des zones définies de manière procédurale sur l'image ultrasonore (33) en fonction d'une intersection de suivi de l'image ultrasonore (33) et du volume ultrasonore (42).

7. Système selon la revendication 5, dans lequel la station de travail d'imagerie ultrasonore (32) différencie au moins deux zones définies de manière procédurale visualisées dans l'image ultrasonore (33).

8. Système selon la revendication 7, dans lequel une différenciation des au moins deux zones définies de manière procédurale visualisées dans l'image échographique (33) comprend au moins l'un d'un codage couleur, d'un étiquetage de texte et d'un retour audio.

9. Procédé mis en œuvre par ordinateur pour la visualisation automatique de zones, le procédé comprenant:
la génération d'un volume ultrasonore (42) d'une prostate dérivé d'un balayage ultrasonore d'une région anatomique; et
le suivi d'un étiquetage de zones de la prostate définies de manière procédurale à l'intérieur du volume ultrasonore (42) dérivé d'un modèle de volume ultrasonore (41) de la prostate étiqueté avec les zones définies de manière procédurale,
dans lequel les zones définies de manière procédurale à l'intérieur du volume ultrasonore (42) facilitent une visualisation guidée par ultrasons de la prostate selon un schéma d'échantillonnage par biopsie, qui est ajouté au modèle volumique ultrasonore de la prostate (41) en identifiant un certain nombre de N emplacements dans la prostate comme objets géométriques qui servent à guider un échantillonnage systématique de la prostate.

10. Procédé selon la revendication 9, dans lequel le suivi de l'étiquetage des zones de la prostate définies de manière procédurale dans le volume échographique (42) comprend:
la mise en correspondance du modèle de volume ultrasonore (41) avec une génération suivie du volume ultrasonore (42).

11. Procédé selon la revendication 9, dans lequel le suivi de l'étiquetage des zones de la prostate définies de manière procédurale dans le volume échographique (42) comprend:
la mise en correspondance du modèle de volume ultrasonore (41) avec une segmentation de la prostate à partir d'une génération suivie du volume ultrasonore (42).

12. Procédé selon la revendication 9, dans lequel le modèle de volume ultrasonore (41) est un modèle de subdivision géométrique d'une segmentation de la prostate dans le volume ultrasonore (42).

13. Procédé selon la revendication 9, comprenant en outre:
la génération d'une image ultrasonore (33) en réponse à une sonde ultrasonore (31) guidant une visualisation d'au moins une des zones de la prostate définies de manière procédurale,
dans lequel l'image ultrasonore (33) visualise l'au moins une des zones définies de manière procédurale dérivée du volume ultrasonore étiqueté par zone (42).

14. Procédé selon la revendication 13,
dans lequel la génération de l'image échographique (33) est suivie à l'intérieur de l'espace du patient et
dans lequel l'une au moins des zones définies de manière procédurale est étiquetée par zone sur l'image échographique (33) en fonction d'une intersection de suivi de l'image échographique (33) et du volume échographique (42).

15. Procédé selon la revendication 14, dans lequel au moins deux zones définies de manière procédurale sont différenciées dans l'image échographique (33).
